# EUROPEAN PATENT SPECIFICATION

(11) **EP 3 406 588 B1**
(45) Date of publication and mention of the grant of the patent: **19.08.2020**
(21) Application number: 16886210.0
(22) Date of filing: 20.01.2016
(51) Int. Cl.: C07C 51/56, C07C 53/12, B01J 31/02, C07C 45/89, C07C 49/92, C07C 45/90, C07C 49/90

(54) **METHOD FOR PRODUCING KETENE DERIVATIVE**
VERFAHREN ZUR HERSTELLUNG VON KETENDERIVAT
PROCÉDÉ DE PRODUCTION D'UN DÉRIVÉ DE CÉTÈNE

(43) Date of publication of application: 28.11.2018
(73) Proprietor: Daicel Corporation, Osaka-shi, Osaka 530-0011 (JP)
(72) Inventor: SASAKI, Yukihiro, Himeji-shi Hyogo 671-1283 (JP); OKUYAMA, Naoto, Himeji-shi Hyogo 671-1283 (JP); TSUGITA, Nobuhiro, Himeji-shi Hyogo 671-1283 (JP)
(74) Representative: Grünecker Patent- und Rechtsanwälte PartG mbB
(86) International application number: PCT/JP2016/000267
(87) International publication number: WO 2017/125964

(56) References cited:
- GB-A- 763 018
- JP-A- S6 351 348
- JP-A- 2001 513 522
- JP-A- 2002 284 736
- JP-A- 2002 515 470
- US-A- 2 202 046
- US-A- 2 856 426
- US-A- 4 455 439

## Description

### Technical Field

The present invention relates to a method for producing a ketene derivative.

### Background Art

In a method for producing acetic anhydride, generally, a triethyl phosphate dehydration catalyst is initially used to thermally decompose acetic acid to produce ketene (I), and next the resultant compound ketene is caused to react with acetic acid to produce acetic anhydride (II). Additionally, PTL 1 describes a method of using triethyl phosphate as a catalyst to thermally decompose acetic acid under reduced pressure to produce ketene, and the fact that a gas produced by the thermal decomposition is cooled, and in this step, water, an unreacted fraction of acetic acid, and acetic anhydride are condensed. PTL 2 describes the use of diammonium phosphate as a catalyst. PTL 3 describes the use of phosphoric acid as a catalyst.

A method as described above has widely been performed, in which a phosphorus compound that may be of various types is used as a catalyst to thermally decompose acetic acid to produce ketene, and then acetic anhydride, which is a ketene derivative, is produced from the compound ketene. Various investigations have been made also about the handling of a condensed liquid containing water, an unreacted fraction of acetic acid, acetic anhydride, and others, these contained components being yielded by condensing a thermal decomposition gas produced by the thermal decomposition in the step of cooling this thermal decomposition gas. As a handling of the condensed liquid containing the unreacted fraction of acetic acid, acetic anhydride, and the others, investigations have been made about an effective collection and reuse of the unreacted fraction of acetic acid, and acetic anhydride. The condensed liquid also contains the phosphorus compound, which may be of various types. Thus, from the viewpoint of decreasing a load onto the environment, investigations have been made about the matter that when the phosphorus compound, which may be of various types, is discharged as a wastewater from a plant, the discharge volume thereof is decreased.

As a method for collecting acetic acid and acetic anhydride in the condensed liquid, examples described below are given. As a method for collecting acetic acid, PTL 2 describes a method of supplying an esterifying reactor a condensed liquid containing acetic acid and acetic anhydride, and causing acetic acid in the condensed liquid to react with an alcohol to prepare an acetic ester. Next, as a method for collecting acetic anhydride, PTL 4 describes a method of bringing an aqueous solution containing acetic anhydride and acetic acid into contact with one or more solvents selected from hydrophobic organic solvents, ketones, and esters.

Moreover, as a method in which when a phosphorus compound that may be of various types in the condensed liquid is discharged as a wastewater, known are activated sludge treatment, condensing and precipitating treatment, and other methods. As a method for decreasing and removing a phosphorus compound contained in a wastewater in a process for producing acetic anhydride, PTL 5 describes a method of adding, to the wastewater, a polyvalent metal salt to adjust the pH level thereof to precipitate and remove the compound. GB 763,018 discloses the catalytic pyrolysis of acetic acid for producing ketene. The pyrolysis catalysts employed in the reaction are triethyl phosphate or other volatilisable phosphates which are neutralised once the reaction is completed.

### Citation List

### Patent Literatures

PTL 1: USP No. 4455439
PTL 2: Japanese Patent No. 5390770
PTL 3: JP 2001-513522 A
PTL4: JP H06-080023 B
PTL 5: JP 2014-0527905 A

### Summary of Invention

### Technical Problem

The above has described methods of using a phosphorus compound that may be of various types as a catalyst used in the production of ketene. However, in each of these methods, organic compounds such as acetic acid and acetic anhydride in the condensed liquid are collected, and the phosphorus compound in the condensed liquid is removed from the system of a process for the production. In a removing method therefor, the organic compounds in the condensed liquid are collected, and subsequently the phosphorus compound contained in the remaining wastewater is subjected to, for example, activated sludge treatment, or condensing and precipitating treatment to be removed. Additionally, the concentration of phosphorus is checked, and then the wastewater is disposed of.

Costs are required for such a treatment of a wastewater containing a phosphorus compound, and further it is difficult to decompose and remove, in particular, an organic phosphorus compound, out of phosphorus compounds, also by biodegrading treatment, or condensing and precipitating treatment. Thus, a limitation is imposed onto the removal quantity of the phosphorus compound. Furthermore, it is feared that phosphorus compounds will be exhausted in the future. For this reason, in each of such methods, it is required to decrease the consumption quality of a phosphorus compound and make effective use of the phosphorus compound. Thus, it is necessary to develop a method, for producing ketene and a ketene derivative, that decreases the discharge quantity of phosphorus compounds to be small in load onto the environment. An object of the present invention is to provide a method for producing a ketene derivative which decreases the consumption quality of a phosphorus compound, and the discharge quantity of the phosphorus compound into the environment.

### Solution to Problem

The present inventions relates to a method for producing a ketene derivative, including a step (i) of conducting thermal decomposition reaction of acetic acid in a presence of a phosphorus-containing catalyst in a reactor to produce a thermal decomposition gas containing ketene, a step (ii) of cooling the thermal decomposition gas to be separated into a gaseous component containing ketene, and a condensed liquid containing a phosphorus compound (a), and a step (iii) of causing the ketene to react with a different organic compound having a carboxyl group, a hydroxyl group, or an amino group to produce an acetylated compound in which the carboxyl group, the hydroxyl group ,or the amino group is acetylated, in which the step (i) includes: conducting the thermal decomposition reaction while supplying, into the reactor, the condensed liquid containing the phosphorus compound (a) or a concentrated liquid of the condensed liquid containing the phosphorus compound (a), and wherein the phosphorus compound (a) is an oxo acid of phosphorus, an oxo acid of any alkylated phosphorus, or a salt of phosphoric acid.

It is preferred that in the step (i), by concentrating the phosphorus compound (a) contained in the condensed liquid, the condensed liquid is turned to the concentrated liquid, and subsequently the concentrated liquid is supplied into the reactor.

It is preferred that a concentration of the phosphorus compound (a) contained in the concentrated liquid is from 5 to 20 times a concentration of the phosphorus compound (a) contained in the condensed liquid.

It is preferred that a method for concentrating the phosphorus compound (a) contained in the condensed liquid is a method selected from the group consisting of heating evaporation, membrane separation, and electrochemical treatment.

It is preferred that in the step (i), a liquid obtained by adding a phosphorus compound (b) to the concentrated liquid is supplied into the reactor.

It is preferred that a ratio by mole of the compound acetic acid to the phosphorus-containing catalyst is from 100 to 2,000.

### Advantageous Effects of Invention

The method of the present invention for producing a ketene derivative makes it possible to decrease the consumption quantity of a phosphorus compound and the discharge quantity of the phosphorus compound into the environment.

### Brief Description of Drawing

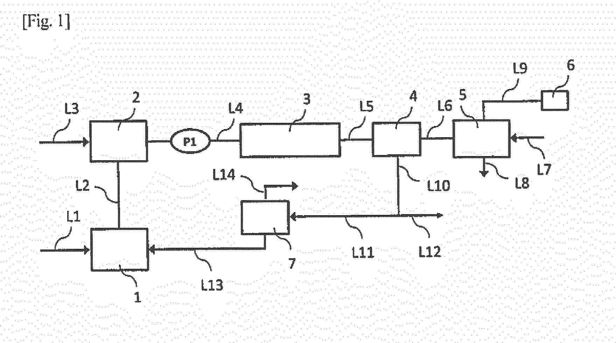

Fig. 1 is a flowchart demonstrating an example of a method for producing ketene and a ketene derivative.

### Description of Embodiments

Hereinafter, an example of a preferred embodiment will be specifically described. The method in the present disclosure for producing a ketene derivative includes a step (i) of conducting thermal decomposition reaction of acetic acid in a presence of a phosphorus-containing catalyst in a reactor to produce a thermal decomposition gas containing ketene, a step (ii) of cooling the thermal decomposition gas to be separated into a gaseous component containing ketene, and a condensed liquid containing a phosphorus compound (a), and a step (iii) of causing the ketene to react with a different organic compound having a carboxyl group, a hydroxyl group, or an amino group to produce a ketene derivative. In this method, the step (i) includes conducting the thermal decomposition reaction while supplying, into the reactor, the condensed liquid containing the phosphorus compound (a) or a concentrated liquid of the condensed liquid.

### [Thermal Decomposition Gas Producing Step (i)]

A description will be made about a procedure and others of the step (i) of conducting thermal decomposition reaction of acetic acid in a presence of a phosphorus-containing catalyst in a reactor to produce a thermal decomposition gas containing ketene (hereinafter, the step may be referred to as the thermal decomposition gas producing step (i)).

The phosphorus-containing catalyst used in the thermal decomposition gas producing step (i) is not particularly limited as far as the catalyst is a catalyst which contains phosphorus to produce ketene by thermal decomposition reaction of acetic acid. According to the present invention,oxo acids of phosphorus, oxo acids of any alkylated phosphorus, and salts of phosphoric acid are used. Examples of the oxo acids of phosphorus include phosphoric acid, and pyrophosphoric acid. Examples of oxo acids of the alkylated phosphorus include dimethylphosphinic acid and triethylphosphoric acid. Examples of the salts of phosphoric acid include diammonium hydrogenphosphate. However, the acids and the salts are not limited to these examples. Such phosphorus-containing catalysts may be used singly or in any combination of two or more thereof. The phosphorus-containing catalyst(s) may contain a phosphorus based compound having no catalytic effect. When two or more of the phosphorus-containing catalysts are combined with each other, the combination preferably contains diammonium hydrogenphosphate from the viewpoint of the catalytic efficiency thereof. The phosphorus-containing catalyst(s) may (each) be a mixed solution in which a solvent such as water or acetic acid is used.

In the thermal decomposition gas producing step (i), specifically, a phosphorus-containing catalyst dissolved in a solvent such as water or acetic acid is added to acetic acid, and further the resultant is introduced into a reactor (reaction furnace) preheated into high-temperature and reduced-pressure conditions, or a phosphorus-containing catalyst dissolved in a solvent such as water or acetic acid, and preheated acetic acid are introduced into a reactor (rector furnace). This manner makes it possible to start and advance thermal decomposition reaction of acetic acid to produce a thermal decomposition gas containing ketene.

The thermal decomposition reaction of acetic acid is preferably conducted under high-temperature and reduced-pressure conditions. The high conditions may be, for example, conditions of about 700 to 725°C temperature. The reduced-pressure conditions are, for example, conditions of a driving operation pressure of about 30 kPa (A), and the pressure may be about 26 kPa (A).

After the reactor is made into the high-temperature and reduced-pressure conditions, acetic acid, which is a raw material, and the phosphorus-containing catalyst, which is a catalyst, are supplied into the reactor so that the thermal decomposition reaction advances to generate the thermal decomposition gas, which contains ketene. The driving operation pressure is the pressure of the inside of the reactor at its outlet moiety.

It is preferred to heat (preheat) acetic acid, which is a raw material, into the range of 100 to 700°C by means of a preheater, and then supply the heated acid into the reactor. It is allowable to use a method of heating the phosphorus-containing catalyst together with acetic acid by means of the preheater, and then supply the two components into the reactor, or a method of supplying acetic acid into the reactor, and then supply the phosphorus-containing catalyst directly into the reactor. In the thermal decomposition gas producing step (i), the ratio by mole of acetic acid to the phosphorus-containing catalyst is, for example, from 100 to 2,000, preferably from 300 to 1,000. The adjustment of the ratio of acetic acid to the phosphorus-containing catalyst can be attained by adjusting the concentration of the phosphorus-containing catalyst in the solution of acetic acid. The molar quantity of the phosphorus-containing catalyst is the total of one phosphorus compound, or two or more various phosphorus compounds therein.

### [Separating Step (ii)]

Through the thermal decomposition gas producing step (i), acetic acid simultaneously gives ketene and water. Additionally, an unreacted fraction of acetic acid may be present. All of these substances are in a gas state in the thermal decomposition gas producing step (i), and are present as a thermal decomposition gas. Thus, by cooling this thermal decomposition gas into an appropriate temperature, this gas can be separated to a gaseous component containing ketene, and a condensed liquid containing water, acetic acid, and one or more phosphorus compounds (a). The following will describe a procedure and others of the step (ii) of separating the gas to the gaseous component containing ketene, and the condensed liquid containing the phosphorus compound(s) (a) (hereinafter, this step may be referred to as the separating step (ii)).

About the cooling of the thermal decomposition gas in this step, the cooling temperature is not particularly limited as far as the temperature is a temperature capable of separating the gas to the gaseous component containing ketene, and the condensed liquid containing water, acetic acid, and the phosphorus compound(s) (a). It is preferred to adjust the temperature of the gas into the range of -10 to 220°C from the viewpoint of the use quantity of energy and the viewpoint of the introduction of ketene with a high purity in the step (iii) of causing the ketene to react with a different organic compound to produce a ketene derivative.

For cooling the thermal decomposition gas, a heat exchanger (gas cooling device) may be used. The heat exchanger may be singly used, or plural arranged heat exchangers may be used. The plural heat exchangers may be, for example, two, three, four or five heat exchangers. The case where the plural heat exchangers are arranged is preferred since, in this case, condensed liquid fractions from all the heat exchangers may be used as the phosphorus-containing catalyst, and additionally from the heat exchangers, any one or more heat exchangers may be selected, and its/their condensed liquid fraction(s) can be selectively used as the phosphorus-containing catalyst. The selection of the heat exchanger(s) from which the condensed liquid fraction(s) is/are to be collected makes it possible to prevent the collected condensed liquid from containing carbon and nonvolatile components originating from the ketene decomposing furnace (the reactor in the thermal decomposition gas producing step (i)) and originating from coking in the furnace, and adjust the concentration of the phosphorus compound(s) contained in the condensed liquid.

A description will be made about an example of the case of using five heat exchangers. First and second ones of the heat exchangers are filled with cold water, and the three remnants are filled with cold brine of -10°C temperature. The five heat exchangers are the same as each other, and each have a length of 2500 mm, and a diameter of 700 mm. This has 290 tubes each having an inside diameter of 25 mm, and a total inside surface area of 285 m². The total volume of the five heat exchangers which include their uppers and separations is 3.5 m³, and the proportion of the surface to the volume is 81.5 m⁻¹. When the thermal decomposition gas generated in the thermal decomposition gas producing step (i) is introduced into the heat exchangers, the thermal decomposition gas is separated into a gaseous component and a liquid component in the first heat exchanger. The upper gaseous component is introduced into the second heat exchanger, and the liquid component on the bottom thereof is collected into a column for liquid-component-collection. Subsequently, a gaseous component in the upper of the second heat exchanger, the third heat exchanger, and the fourth heat exchanger are introduced, respectively, into the third heat exchanger, the fourth heat exchanger, and the fifth heat exchanger in order. Respective liquid components on the bottoms of these heat exchangers are collected onto the column for liquid-component-collection. From the upper of the fifth heat exchanger, a gaseous component is collected. This procedure makes it possible to separate the thermal decomposition gas into the gaseous component and the condensed liquid, which is a liquid component. About the condensed liquid of the phosphorus compound(s) in the present invention, it is allowable to collect the liquid components on the bottoms of all the heat exchangers, or collect the liquid component(s) on the bottom of any one of the first to fourth heat exchangers, or on the bottoms of two or more of the same heat exchangers. The liquid components on the bottoms of the first and second heat exchangers may be collected since the phosphorus compound concentration therein is particularly high.

### [Ketene Derivative Producing Step (iii)]

A description will be made about a procedure and others of the step (iii) of causing the ketene to react with a different organic compound having a carboxyl group, a hydroxyl group, or an amino group to produce a ketene derivative.

When the different organic compound has a functional group such as a carboxyl group, a hydroxyl group, or an amino group, the organic compound reacts with ketene to produce a ketene derivative. This reaction can be conducted, for example, by introducing ketene gas into an absorption tower, into which the different organic compound has been supplied.

When the different organic compound has a hydroxyl group or an amino group, ketene reacts with this functional group to acetylate the different organic compound. When, for example, acetic acid is used as the different organic compound, acetic acid and ketene react with each other to produce acetic anhydride, which is a ketene derivative. The method for the reaction may be a method of causing acetic acid to remain, and bubbling ketene gas thereinto. The reaction method may be a method of bringing acetic acid and ketene into countercurrent contact with each other in a packed tower. In this case, in order to remove heat of the reaction between acetic acid and ketene, the temperature of the inside of the system is preferably from 20 to 70°C, and the pressure inside the system is preferably from 0 to 30 kPa (A). Specifically, when acetic acid and ketene are caused to react with each other in the absorption tower adjusted to pressure-reduced conditions, acetic anhydride can be obtained.

The resultant ketene derivative may be optionally purified by a known ordinary method such as extraction, distillation or ozone treatment. When the ketene derivative is, for example, acetic anhydride, a separating method based on distillation may be used.

### [Supply of Condensed Liquid Containing Phosphorus Compound(s) (a), or Concentrated Liquid Thereof to Reactor in Step (i)]

In the method in the present disclosure for producing a ketene derivative, the condensed liquid containing the phosphorus compound(s) (a) or a concentrated liquid thereof is supplied into the reactor in the thermal decomposition gas producing step (i). The phosphorus compound(s) (a) can be reused as a phosphorus-containing catalyst for the thermal decomposition reaction of acetic acid. In this case, as the phosphorus-containing catalyst for the thermal decomposition reaction of acetic acid, the phosphorus compound(s) (a) is/are usable while the compound(s) is/are kept in the state of the condensed liquid yielded through the separating step (ii), or by supplying a concentrated liquid of the condensed liquid into the reactor, in which the thermal decomposition reaction of acetic acid is conducted. In the present disclosure, the phosphorus compound(s) (a) denote(s) a phosphorus compound that may be of various types, or various phosphorus compounds, the compound or compounds being contained in the condensed liquid or the concentrated liquid.

Dependently on the phosphorus compound(s) used as the phosphorus-containing catalyst, the phosphorus compound(s) (a) may be yielded as a mixture of various phosphorus compounds since the phosphorus-containing catalyst is decomposed, which follows the thermal decomposition reaction of acetic acid. In the case of using, as the phosphorus-containing catalyst, for example, diammonium hydrogenphosphate, the phosphorus compound(s) (a) is/are a mixture of phosphoric acid, dimethylphosphinic acid, and other phosphorus compounds.

It has been hitherto considered that it is possible to use only diammonium hydrogenphosphate (DAP), triethyl phosphate (TEP) or the like as the phosphorus-containing catalyst used in a ketene decomposing furnace. The inventors have found out that even in the case of using, as a catalyst, a phosphorus compound, such as dimethylphosphinic acid (DMPH), contained in dilute acetic acid (chiller condensed liquid) produced as a byproduct, the efficiency of the catalyst as the whole thereof is not damaged. Hitherto, the phosphorus compound(s) (a), such as dimethylphosphinic acid (DMPH), has/have been regarded as one or ones having no catalytic function. Thus, the phosphorus compound(s) (a) contained in the condensed liquid is/are disposed of as a wastewater.

Even when the phosphorus compound(s) (a) is/are used as the phosphorus-containing catalyst in the thermal decomposition gas producing step (i), substantially the same ketene yield can be obtained as when a phosphorus-containing catalyst that has not yet been related to the thermal decomposition reaction of acetic acid is used. These two cases have substantially the same catalytic function. For this reason, by substituting the phosphorus-containing catalyst in the thermal decomposition gas producing step (i) partially with the phosphorus compound(s) (a), decreases can be attained in the consumption quality of the phosphorus compound(s) as the phosphorus-containing catalyst, and in the discharge quantity of the compound(s) to the environment in the thermal decomposition gas producing step (i). The consumption quality of the phosphorus compound(s) denotes the proportion of the molar number of one or various phosphorus compounds charged newly into the reactor to the molar number of acetic acid supplied into the reactor. For reference, the decreasing proportion of the discharge quantity of the phosphorus compound(s) is substantially equal to that of the consumption quantity of the phosphorus compound(s).

### (Preparation of Concentrated Liquid)

The phosphorus compound(s) (a) contained in the condensed liquid is/are usable as a catalyst in the thermal decomposition gas producing step (i) while the state of the obtained condensed liquid is kept. However, it is preferred to concentrate the phosphorus compound(s) (a) contained in the condensed liquid into a concentrated liquid, and then supply this concentrated liquid, in the state that the concentration of the phosphorus compound(s) has been made high, into the reactor in the thermal decomposition gas producing step (i). Furthermore, the concentrated liquid may be further concentrated into a solid of the phosphorus compound(s). In this case, it is preferred to mix the solid with water into an aqueous solution state, and use the mixture in this state as a catalyst. In other words, any composition that contains the phosphorus compound(s) (a) is usable as the phosphorus-containing catalyst in the thermal decomposition gas producing step (i).

The following will describe a procedure of making the condensed liquid into a concentrated liquid by concentrating the phosphorus compound(s) (a) contained in the condensed liquid. The method for concentrating the phosphorus compound(s) (a) contained in the condensed liquid may be, for example, a known ordinary method such as heating evaporation, membrane separation, or electrochemical treatment, or a method in which two or more of these methods may be combined with each other. It is sufficient that such a method is used to concentrate the phosphorus compound(s) (a) to an arbitrarily-selected concentration ratio. The composition of the condensed liquid is composed mainly of water, acetic acid, and the phosphorus compound(s) (a); thus, it is preferred to attain the concentration by heating evaporation, out of these methods, to remove water, and further avoid the effect of carbon and other nonvolatile components contained in the condensed liquid, these components originating from the ketene decomposing furnace, and coking.

When the heating evaporation is performed, the condensed liquid is evaporated and concentrated in an evaporating and concentrating device to remove volatile components, such as acetic acid, together with water to concentrate the condensed liquid. In this way, a concentrated liquid having the phosphorus compound(s) high in concentration can be yielded. The evaporating and concentrating device may be a known device such as an evaporator. The evaporating and concentrating device is a device for evaporating and concentrating a substance under wide pressures such as normal pressure or reduced pressure. The evaporator is a device for evaporating and concentrating a liquid positively under reduced pressure. The concentration phosphorus compound(s) (a) may be further mixed with water, acetic acid, acetic anhydride, and others to be made into a mixed solution thereof with water and acetic acid. Furthermore, from the phosphorus compound(s) (a), volatile components, such as acetic acid, together with water may also be removed to make the compound(s) (a) into a solid that hardly contains any volatile component.

When the membrane separation is performed, the phosphorus compound(s) high in concentration can be collected by separation through an RO (reverse osmotic membrane filtration) membrane. A device for the membrane separation may be a hollow fiber type RO membrane.

When the electrochemical treatment is conducted, the phosphorus compound(s) high in concentration can be collected by separation based on electroosmosis. A device for the separation by electroosmosis may be an electroosmotic dehydrator.

The concentration ratio of the phosphorus compound(s) (a) contained in the concentrated liquid is selectable at will. The target concentration of the phosphorus compound(s) (a) is set to in a range preferably from 1 to 100 times, more preferably from 2 to 50 times, even more preferably from 5 to 20 times the concentration of the phosphorus compound(s) (a) contained in the condensed liquid. This is because this case makes the phosphorus compound(s) (a) excellent in catalytic power when the phosphorus compound(s) (a) is/are used as the phosphorus-containing catalyst in the thermal decomposition gas producing step (i). Also when the evaporating and concentrating device is used, it is sufficient for the concentration to be set into any one of the above-mentioned concentration ratio ranges. The concentration ratio is preferably into a range from 5 to 20. This is because this case makes it possible to produce a ketene derivative stably in the state of making the ratio by mole between acetic acid, water, and phosphorus constant so that the concentrated liquid is excellent in operability.

The concentration ratio of the phosphorus compound(s) (a) is obtained using, as a standard thereof, the total concentration of the phosphorus compound(s) (a), which may (each) be of various types and is/are contained in the solution. The total concentration of the phosphorus compound(s) (a), which may (each) be of various types, is measurable by, for example, an ICP emission spectroscopic analysis prescribed in JIS K 0116, or ion chromatography prescribed in JIS K 0127.

When the phosphorus compound(s) (a) contained in the condensed liquid or the concentrated liquid thereof is/are supplied into the reactor in the thermal decomposition gas producing step (i), it is preferred for advancing the thermal decomposition reaction of acetic acid more stably that one or more phosphorus compound(s) (b), acetic acid, water and others are appropriately added to the condensed liquid or the concentrated liquid to adjust the concentration of each of these components to a predetermined concentration. In the present disclosure, the phosphorus compound(s) (b) denote(s) one or more phosphorus compounds that may (each) be of various types and are added newly to the condensed liquid or the concentrated liquid thereof.

Examples of the phosphorus compound(s) (b) preferably include compounds given as the examples of the phosphorus-containing catalyst(s) used in the thermal decomposition gas producing step (i). Usable examples thereof include oxo acids of phosphorus, oxo acids of any alkylated phosphorus, and salts of phosphoric acid. Examples of the oxo acids of phosphorus include phosphoric acid, and pyrophosphoric acid. Examples of oxo acids of the alkylated phosphorus include dimethylphosphinic acid, and triethylphosphoric acid. Examples of the salts of phosphoric acid include diammonium hydrogenphosphate. However, the phosphorus compound(s) (b) is/are not limited to these examples. These compounds may be used singly, or in any combination of two or more thereof. It is preferred to use, out of these examples, diammonium hydrogenphosphate because of a high activity thereof and economy. It is also preferred to use a mixture of diammonium hydrogenphosphate and a phosphorus compound that is a variant thereof.

In any one of the case of adding the phosphorus compound(s) (b), acetic acid, water, and others to the condensed liquid or the concentrated liquid thereof, and the case of adding none of these components thereto, the above-mentioned predetermined concentration of each of the components in the condensed liquid or the concentrated liquid is as follows: the concentration of acetic acid is preferably from 85 to 99.9% by weight, more preferably from 90 to 99% by weight; and the concentration of the total of the phosphorus compound(s), which may (each) be of various types, is preferably from 0.1 to 1 % by weight, more preferably from 0.2 to 0.7% by weight.

When the thermal decomposition gas producing step (i) is industrially performed while the condensed liquid containing the phosphorus compound(s) (a), or the concentrated liquid thereof is supplied into the reactor, it is preferred, for making the management of the operation easy, that the composition of the condensed liquid containing the phosphorus compound(s) (a), or the concentrated liquid is adjusted to be made as constant as possible. This is because when the composition of the condensed liquid containing the phosphorus compound(s) (a) or the concentrated liquid is always varied, the width or frequency of the adjustment of the composition becomes large to make a load onto the operation large.

It is preferred that the adjustment of the concentration of each of the components contained in the condensed liquid or the concentrated liquid is made in a catalyst preparing tank located before the reactor. The catalyst preparing tank is selectable from known ordinary devices such as a batch type stirrer, a continuous stirrer, a static mixer, and others, or a device in which two or more of these devices are combined with each other. However, the catalyst preparing tank is not particularly limited.

About the condensed liquid, a fraction thereof that is other than a fraction thereof which has been turned to the concentrated liquid by the concentration of the phosphorus compound(s) (a) hardly contains any phosphorus compound, and contains water and organic compounds such as acetic acid, which are volatile components. The organic compounds can be collected and used by a known ordinary method such as extraction or distillation. After the collection of the organic compounds, the condensed liquid contains other predetermined organic compounds, the phosphorus compound(s), and other inorganic compounds. It is therefore necessary to conduct wastewater treatment to decrease a load onto the environment. In the wastewater treatment, it is preferred to decompose and remove the organic compounds, the phosphorus compound(s), and the other inorganic compounds in the aqueous solution by a known ordinary method such as activated sludge treatment or condensing and precipitating treatment, so as to decrease the respective concentrations of the compounds, and subsequently discharge the removed compounds. Out of the organic compounds, acetic acid is contained in the largest proportion. Thus, acetic acid can be collected by an ordinarily used technique for collecting acetic acid from a dilute acetic acid solution. In short, for example, a method described in JP S57-197240 may be used. This acetic acid collecting method is a method of using a mixed solvent to extract acetic acid from an aqueous solution, and separating acetic acid from the extraction liquid by distillation, characterized in that the mixed solvent is an ethyl acetate/diisobutyl ketone based solvent. The proportion of ethyl acetate in the mixed solvent is usually from 5 to 50% by weight, in particular from 20 to 30% by weight.

When the ethyl acetate/diisobutyl ketone based mixed solvent is used, it is sufficient for only water and ethyl acetate to be evaporated in a dehydrating tower. Diisobutyl ketone is taken out together with acetic acid toward the cooker, and in an acetic acid collecting tower, acetic acid is separated from the taken-out liquid. Thereafter, the ketone is recycled as it is to be used. The treated raw liquid is charged into an extracting tower, and the mixed solvent is charged into the extracting tower through a different line. The extraction liquid contains saturated water together with acetic acid; thus, the liquid is initially charged into a dehydrating tower. In the dehydrating tower, ethyl acetate, and a water fraction saturated and dissolved are removed from the top of the tower, and a mixture of diisobutyl ketone and acetic acid is taken out from the bottom of the tower. In this way, water and acetic acid are separated from each other. Thereafter, the dehydrating tower liquid is charged into an acetic acid collecting tower, and then acetic acid is collected from the top of the tower. From the bottom of the tower, diisobutyl ketone is taken out. The ketone is mixed with ethyl acetate taken out from the dehydrating tower top, and the mixture is recycled into the extracting tower to be used as a circulated extracting solvent. The treated wastewater, from which acetic acid has been extracted in the extracting tower, is charged into a solvent collecting tower. From the top of the tower, the solvent dissolved in a slight quantity in the wastewater is collected. The collected solvent is returned into the circulated extracting solvent.

Hereinafter, an example of the embodiment in the present disclosure will be described with reference to the drawing. Fig. 1 is a flowchart demonstrating an example of a method for producing ketene, and a ketene derivative. Reference number 1 represents a catalyst preparing tank. In the tank is stored a catalyst in the thermal decomposition gas producing step (i) based on thermal decomposition reaction of acetic acid. In the catalyst preparing tank 1, the catalyst is stored, and further one or more phosphorus compounds (b), acetic acid, water and others can be appropriately added to a condensed liquid supplied from a heat exchanger 4, which will be detailed later, or to a concentrated liquid supplied from a concentrating device 7 to adjust each of the components into a target concentration. The phosphorus compound(s) (b), acetic acid, water and the others to be added may be supplied through a line L1 into the catalyst preparing tank 1. The condensed liquid may be directly supplied from the heat exchanger 4 without being passed through the concentrating device 7. It is preferred to pass the condensed liquid into the concentrating device 7 to be made to a concentrated liquid, and then pass the concentrated liquid through a line L13 to be supplied into the catalyst preparing tank 1.

Reference number 2 represents a raw material tank. In the tank, acetic acid to be supplied into a reactor 3, which will be detailed later, is stored. Acetic acid can be supplied through a line L3 into the raw material tank 2. Before the supply of acetic acid into the reactor 3, it is allowable to blend, with acetic acid, one or more phosphorus compounds supplied beforehand through a line L2 from the catalyst preparing tank 1 in the raw material tank 2.

Reference number 3 represents the reactor. The reactor receives acetic acid from the raw material tank 2 to conduct thermal decomposition reaction of acetic acid, using the phosphorus compound(s) as a catalyst. As the method for supplying the phosphorus compound(s) into the reactor 3, any method is selectable from a method of supplying the compound(s) via the raw material tank 2, and a method of supplying the compound(s) from the catalyst preparing tank 1 directly to the reactor 3. The reactor 3 also includes a preheater. In this preheater, before the thermal decomposition reaction, acetic acid and the catalyst are heated to a temperature of about 100 to 700°C, and may be heated to about 725 °C according to circumstances. In this way, the advance of the thermal decomposition reaction can be promoted.

Reference number PI represents a charging pump. The pump makes it possible to supply a raw material liquid containing acetic acid and the catalyst at a constant supply rate from the raw material tank 2 through a line L4 into the reactor 3. The charging pump P1 is a booster pump.

Reference number 4 represents the heat exchanger (gas cooler). The heat exchanger receives a thermal decomposition gas generated by the thermal decomposition reaction in the reactor 3 through a line L5, and cools the thermal decomposition gas to separate this gas into a gaseous component and a condensed liquid, which is a liquid component. The heat exchanger 4 is made of, for example, a water cooler, a cold water cooler, and a brain cooler. The high-temperature gas is cooled to a temperature near 0°C so that a large temperature difference is generated. It is therefore preferred to lower the temperature of the gas step by step, or lower the pressure step by step. For reference, the condensed liquid contains water, an unreacted fraction of acetic acid, the added phosphorus compounds, and various phosphorus compounds generated following the thermal decomposition reaction of acetic acid.

Reference number 5 represents an acetic acid trap. The trap receives ketene, which is a gaseous component, through a line L6 from the heat exchanger 4, and causes this compound ketene to react with acetic acid to produce acetic anhydride. Acetic acid can be supplied through a line L7, and produced acetic anhydride can be discharged through a line L8. The acetic acid trap 5 is, for example, a packed tower, and can obtain acetic anhydride by causing acetic acid and ketene to react with each other by bringing a ketene gas introduced from the tower bottom into countercurrent contact with acetic acid caused to flow down from the tower top.

Reference number 6 represents a vacuum pump, and is a member for reducing the insides of the reactor 3, the heat exchanger 4 (gas cooler), and the acetic acid trap 5 in pressure.

Reference number 7 represents the concentrating device, and is a member for concentrating the condensed liquid containing the phosphorus compound(s) into any concentration to prepare a concentrated liquid. From the heat exchanger 4, the condensed liquid, which is a liquid component, is supplied through a line L10 and a line L11 to the concentrating device 7. About the condensed liquid, a fraction thereof that is other than a fraction thereof which has been turned to the concentrated liquid by the concentration of the phosphorus compound(s) (a) is passed through a line L14 to be separated into organic compounds, and a wastewater containing the phosphorus compound(s) and others. The separated organic compounds are collected to be used, and the wastewater containing the phosphorus compound(s) and the others is subjected to activated sludge treatment and condensing and precipitating treatment, so that the phosphorus compound concentration in the wastewater is decreased. Thereafter, the wastewater is discharged into the environment.

When the volume of the condensed liquid generated in the heat exchanger 4 is not entirely supplied into the concentrating device 7, a fraction of the condensed liquid that is not supplied into the concentrating device 7 is passed not through a Line L11 but through a line L12 to be separated into organic compounds, and a wastewater containing the phosphorus compound(s), and others. The separated organic compounds are collected to be used, and the wastewater containing the phosphorus compound(s) and the others is subjected to activated sludge treatment and condensing and precipitating treatment, so that the phosphorus compound concentration in the wastewater is decreased. Thereafter, the wastewater is discharged into the environment.

The concentrated liquid yielded in the concentrating device 7 is supplied through a line L13 into the catalyst preparing tank 1 to be usable as a catalyst in the thermal decomposition gas producing step (i) based on thermal decomposition reaction of acetic acid since the concentrated liquid has a catalytic power for the thermal decomposition reaction of acetic acid in the reactor 3, that is, a ketene-producing reaction.

The method in the present disclosure for producing a ketene derivative makes it possible to use the following again as a catalyst for thermal decomposition reaction of acetic acid: the condensed liquid containing one or more phosphorus compounds used at least once as the catalyst for the thermal decomposition reaction of acetic acid; or the phosphorus compound(s) contained in the concentrated liquid of the condensed liquid. It is therefore possible to decrease the consumption quantity of the phosphorus compound(s) and the discharge quantity of the phosphorus compound(s) into the environment.

### EXAMPLES

Hereinafter, a specific description will be made about a method in the present disclosure for producing a ketene derivative by way of working examples thereof. However, the technical scope of the present invention is never limited by these working examples.

### <Acetic Anhydride Concentration>

The concentration of acetic anhydride can be gained by gas chromatography.

### <Acetic Anhydride Yield>

The yield of acetic anhydride denotes the proportion of the molar number of produced acetic anhydride into that of acetic acid supplied into a reactor.

The yield of acetic anhydride can be obtained by gaining the molar number of acetic acid supplied into the reactor, and that of produced acetic anhydride by the above-mentioned method gas chromatography, and calculating out the proportion of the molar number of produced acetic anhydride into that of acetic acid supplied into the reactor.

### <Use Amount of Phosphorus Compound(s)>

The use quantity of a phosphorus compound is the ratio of the molar number of one or various phosphorus that are supplied into a reactor to that of acetic acid supplied into the reactor.

The use quantity of the phosphorus compound(s) is gained by ion chromatography. Measuring conditions of the ion chromatography are as follows:

| | |
|---|---|
| Device: | DIONEX ICS-2000 |
| Pre-column | AG9-HC |
| Column: | AS9-HC |
| Column temperature: | 40°C |
| Detection: | Electroconductivity detector |
| Eluent; | 9mmol/l(Na₂CO₃) |
| Rate: | 1ml/min |
| Injected quantity: | 100µl |

The ketene and ketene-derivative producing apparatus illustrated in Fig. 1 was used to produce ketene and ketene derivatives.

### (Reference Example 1)

In the catalyst preparing tank 1, 0.32 parts by weight of diammonium hydrogenphosphate were dissolved, as a phosphorus-containing catalyst, into 3.03 parts by weight of water to prepare a phosphorus-containing catalyst solution in water. In the raw material tank 2, 96.65 parts by weight of acetic acid were added to the phosphorus-containing catalyst solution in water, and these were mixed with each other to prepare a raw material liquid. The ratio by mole of acetic acid to the phosphorus-containing catalyst in the raw material liquid was 664. The results are shown in Table 1.

The raw material liquid was supplied into the reactor 3 having a pressure reduced to 26 kPa (A) through the vacuum pump 6 and a temperature of 725°C to advance thermal decomposition reaction of acetic acid. The charging pump PI was used to adjust the supply rate of the raw material liquid into about 20.2 g/h. A thermal decomposition gas discharged from the reactor 3 was rapidly cooled through the heat exchanger 4, which was made of a metal, to produce ketene, which is a gaseous component, and a condensed liquid containing an unreacted fraction of acetic acid, acetic anhydride, water, and phosphorus compounds, which are liquid components.

Ketene, which is a gaseous component, was used, and bubbled into 300 g of acetic acid put in the acetic acid trap 5, which was an absorption tube made of glass, at room temperature to produce acetic anhydride. After about 0.5 hours elapsed, the concentration of acetic anhydride in the acetic acid trap 5 was 2.73% by weight. The acetic anhydride yield was 48.8%. The ratio by mole of the use quantity of the phosphorus compound to that of produced acetic anhydride was 0.003. About the consumption quantity thereof, the ratio by mole was 0.003.

### (Reference Example 2)

In the catalyst preparing tank 1, 0.24 parts by weight of dimethylphosphinic acid were dissolved, as a phosphorus-containing catalyst, into 3.00 parts by weight of water to prepare a phosphorus-containing catalyst solution in water. In the raw material tank 2, 96.76 parts by weight of acetic acid were added to the phosphorus-containing catalyst solution in water, and these were mixed with each other to prepare a raw material liquid. The ratio by mole of acetic acid to the phosphorus-containing catalyst in the raw material liquid was 631.

The raw material liquid was supplied into the reactor 3 having a pressure reduced to 26 kPa (A) through the vacuum pump 6 and a temperature of 725°C to advance thermal decomposition reaction of acetic acid. The charging pump PI was used to adjust the supply rate of the raw material liquid into about 21.2 g/h. A thermal decomposition gas discharged from the reactor 3 in the reactor 3 was rapidly cooled through the heat exchanger 4 to produce ketene, which is a gaseous component, and a condensed liquid containing an unreacted fraction of acetic acid, acetic anhydride, water, and phosphorus compounds, which are liquid components.

Ketene, which is a gaseous component, was used, and bubbled into 300 g of acetic acid put in the acetic acid trap 5 to produce acetic anhydride. After about 0.5 hours elapsed, the concentration of acetic anhydride in the acetic acid trap 5 was 0.76% by weight. The acetic anhydride yield was 12.2%. The ratio by mole of the use quantity of the phosphorus compound to that of produced acetic anhydride was 0.013. About the consumption quantity thereof, the ratio by mole was 0.013. The results are shown in Table 1.

### (Example 1)

An evaporator as the condensing device 7 was used to concentrate the condensed liquid produced in Reference Example 1 at a temperature of 67°C and a pressure of 11 kPa (A) to make the total quantity (weight) of the phosphorus compounds 12 times larger than the original quantity thereof. In this way, a concentrated liquid was prepared.

The composition of the phosphorus compounds (a) contained in the condensed liquid was as follows: phosphoric acid: 0.16% by weight; dimethylphosphinic acid: 0.08% by weight; and other phosphorus compounds: 0.31% by weight. The phosphorus compounds (a) were produced by the decomposition of diammonium hydrogenphosphate used in Reference Example 1, which followed the thermal decomposition reaction of acetic acid. The composition of the phosphorus compounds (a) contained in the concentrated liquid accumulated on the bottom of the concentrating device 7 was as follows: phosphoric acid: 1.90% by weight; dimethylphosphinic acid: 0.88% by weight; and other phosphorus compounds: 3.13% by weight.

Next, in the catalyst preparing tank 1, 3.91 parts by weight of the concentrated liquid were dissolved into 3.01 parts by weight of water to prepare a phosphorus compound (a) solution in water. In the raw material tank 2, 96.76 parts by weight of acetic acid were added to the phosphorus compound (a) solution in water to prepare a raw material liquid. The ratio by mole of acetic acid to the phosphorus-containing catalyst contained in the raw material liquid was 672.

The raw material liquid was supplied into the reactor 3 having a pressure reduced to 26 kPa (A) through the vacuum pump 6 and a temperature of 725°C to advance thermal decomposition reaction of acetic acid. The charging pump PI was used to adjust the supply rate of the raw material liquid to about 20.4 g/h. The heat exchanger 4 was used to rapidly cool a thermal decomposition gas discharged from the reactor 3 to produce ketene, which is a gaseous component, and a condensed liquid containing an unreacted fraction of acetic acid, acetic anhydride, water, and phosphorus compounds, which are liquid components.

Ketene, which is a gaseous component, was used, and bubbled into 300 g of acetic acid put in the acetic acid trap 5 to produce acetic anhydride. After about 0.5 hours elapsed, the concentration of acetic anhydride in the acetic acid trap 5 was 2.74% by weight.

The acetic anhydride yield was 46.5%. The ratio by mole of the use quantity of the phosphorus compounds to that of produced acetic anhydride was 0.003. About the consumption quantity thereof, the ratio by mole was 0. The consumption quantity in Example 1 is a value obtained by calculation without considering the quantity of the phosphorus-containing catalyst used when the condensed liquid was produced in Reference Example 1 as the quantity of various phosphorus compounds charged newly into the reactor. The results are shown in Table 1.

### (Example 2)

In the same manner as in Example 1, an evaporator as the condensing device 7 was used to concentrate the condensed liquid produced in Reference Example 1 to make the concentration thereof 12 times larger. In this way, a concentrated liquid was prepared. In the catalyst preparing tank 1, the following were dissolved into 3.04 parts by weight of water: 1.96 parts by weight of the concentrated liquid; and 0.16 parts by weight of diammonium hydrogenphosphate, this quantity corresponding to a molar quantity equal to the quantity of phosphorus compounds contained in the 1.96 parts by weight of the concentrated liquid. In this way, a phosphorus compound (a) solution in water was prepared. In the raw material tank 2, 96.69 parts by weight of acetic acid were added to the phosphorus compound (a) solution in water to prepare a raw material liquid. The ratio by mole of acetic acid to the phosphorus-containing catalyst contained in the raw material liquid was 675.

The raw material liquid was supplied into the reactor 3 having a pressure reduced to 26 kPa (A) through the vacuum pump 6 and a temperature of 725°C to advance thermal decomposition reaction of acetic acid. The charging pump PI was used to adjust the supply rate of the raw material liquid into about 19.4 g/h. The heat exchanger 4 was used to rapidly cool a thermal decomposition gas discharged from the reactor 3 to produce ketene, which is a gaseous component, and a condensed liquid containing an unreacted fraction of acetic acid, acetic anhydride, water, and phosphorus compounds, which are liquid components.

Ketene, which is a gaseous component, was used, and bubbled into 300 g of acetic acid put in the acetic acid trap 5 to produce acetic anhydride. After about 0.5 hours elapsed, the concentration of acetic anhydride in the acetic acid trap 5 was 2.00% by weight.

The acetic anhydride yield was 40.0%. The ratio by mole of the use quantity of the phosphorus compounds to that of produced acetic anhydride was 0.004. About the consumption quantity thereof, the ratio by mole was 0.002. The phosphorus compounds used in the present example were phosphorus compounds (b) having a molar quantity equal to that of the collected phosphorus compounds (a). Thus, the "use quantity" was 0.004; however, the "consumption quantity" corresponded to 0.002 (= 0.004 × 0.50). The consumption quantity in Example 2 is a value obtained by calculation without considering the quantity of the phosphorus-containing catalyst used when the condensed liquid was produced in Reference Example 1 as the quantity of various phosphorus compounds charged newly into the reactor. The results are shown in Table 1.

**[Table 1]**

| | Phosphorus compound | Ratio [ratio by mole] of acetic acid to phosphorus compound(s) (a) | Acetic anhydride yield [%] | Use quantity [ratio by mole] of phosphorus compound(s) to acetic anhydride | Consumption quantity [ratio by mole] of phosphorus compound(s) to acetic anhydride |
|---|---|---|---|---|---|
| Example 1 | Concentrated liquid having concentration 12 times that of condensed liquid produced in Reference Example 1 | 672 | 46.5 | 0.003 | 0 |
| Example 2 | Concentrated liquid having concentration 12 times that of condensed liquid produced in Reference Example 1, and diammonium hydrogenphosphate | 675 | 40.0 | 0.004 | 0.002 |
| Reference Example 1 | Diammonium hydrogenphosphate | 664 | 48.8 | 0.003 | 0.003 |
| Reference Example 2 | Dimethylphosphinic acid | 631 | 12.2 | 0.013 | 0.013 |

As shown in Table 1, it is understood that even in the case of supplying various phosphorus compounds contained in a condensed liquid produced by thermal decomposition reaction of acetic acid, or a concentrated liquid thereof to a reactor for the thermal decomposition reaction of acetic acid, substantially the same acetic anhydride yield can be obtained as in a case where in thermal decomposition reaction of acetic acid, a phosphorus compound which has not yet been related to any thermal decomposition reaction of acetic acid is used as a catalyst for the thermal decomposition reaction of acetic acid. In other words, it is understood that the various phosphorus compounds contained in the condensed liquid or the concentrated liquid sufficiently have catalytic activity for the thermal decomposition reaction of acetic acid in the thermal decomposition gas producing step (i).

Thus, the following are understood: when various phosphorus compounds contained in the condensed liquid or concentrated liquid are used to produce ketene and a ketene derivative, the consumption quantity of phosphorus compounds charged newly into a process for producing the ketene derivative can be reduced accordingly; and the discharge quantity of phosphorus from the ketene derivative producing process into the environment can be decreased.

### Industrial Applicability

The present invention can decrease the consumption quantity of phosphorus compounds, and is usable for the production of ketene and a ketene derivative which saves resources and which is small in load to the environment.

### Reference Sings List

- 1: Catalyst preparing tank
- 2: Raw material tank
- 3: Reactor
- 4: Heat exchanger (gas cooler)
- 5: Acetic acid trap
- 6: Vacuum trap
- 7: Concentrating device
- PI: Charging pump
- L1 to 14: Lines

## Claims

1. A method for producing a ketene-derived acetylated compound, comprising:
a step (i) of conducting thermal decomposition reaction of acetic acid in the presence of a phosphorus-containing catalyst in a reactor to produce a thermal decomposition gas containing ketene,
a step (ii) of cooling the thermal decomposition gas to be separated into a gaseous component containing ketene, and a condensed liquid containing a phosphorus compound (a), and
a step (iii) of causing the ketene to react with a different organic compound having a carboxyl group, a hydroxyl group, or an amino group to produce an acetylated compound in which the carboxyl group, the hydroxyl group, or the amino group is acetylated,
wherein the step (i) includes: conducting the thermal decomposition reaction while supplying, into the reactor, the condensed liquid containing the phosphorus compound (a) or a concentrated liquid of the condensed liquid containing the phosphorus compound (a), and
wherein the phosphorus compound (a) is an oxo acid of phosphorus, an oxo acid of any alkylated phosphorus, or a salt of phosphoric acid.

2. The method for producing a ketene-derived acetylated compound according to claim 1, wherein in the step (i), by concentrating the phosphorus compound (a) contained in the condensed liquid, the condensed liquid is turned into the concentrated liquid, and subsequently the concentrated liquid is supplied into the reactor.

3. The method for producing a ketene-derived acetylated compound according to claim 2, wherein a concentration of the phosphorus compound (a) contained in the concentrated liquid is from 5 to 20 times a concentration of the phosphorus compound (a) contained in the condensed liquid.

4. The method for producing a ketene-derived acetylated compound according to claim 2 or 3, wherein a method for concentrating the phosphorus compound (a) contained in the condensed liquid is a method selected from the group consisting of heating evaporation, membrane separation, and electrochemical treatment.

5. The method for producing a ketene-derived acetylated compound according to any one of claims 2 to 4, wherein in the step (i), a liquid obtained by adding a phosphorus compound (b) to the concentrated liquid is supplied into the reactor.

6. The method for producing a ketene-derived acetylated compound according to any one of claims 1 to 5, wherein a ratio by mole of the acetic acid to the phosphorus-containing catalyst is from 100 to 2,000.

## Patentansprüche

1. Verfahren zur Herstellung einer von Keten abgeleiteten acetylierten Verbindung, umfassend:
einen Schritt (i) der Durchführung einer thermischen Zersetzungsreaktion von Essigsäure in Gegenwart eines phosphorhaltigen Katalysators in einem Reaktor, um ein thermisches Zersetzungsgas zu erzeugen, das Keten enthält,
einen Schritt (ii) des Kühlens des thermischen Zersetzungsgases, das in eine gasförmige Komponente, die Keten enthält, und eine kondensierte Flüssigkeit, die eine Phosphorverbindung (a) enthält, getrennt werden soll, und
einen Schritt (iii), bei dem das Keten mit einer anderen organischen Verbindung mit einer Carboxylgruppe, einer Hydroxylgruppe oder einer Aminogruppe zur Reaktion gebracht wird, um eine acetylierte Verbindung herzustellen, in der die Carboxylgruppe, die Hydroxylgruppe oder die Aminogruppe acetyliert ist,
wobei der Schritt (i) umfasst: Durchführen der thermischen Zersetzungsreaktion, während in den Reaktor die kondensierte Flüssigkeit, die die Phosphorverbindung (a) enthält, oder eine konzentrierte Flüssigkeit der kondensierten Flüssigkeit, die die Phosphorverbindung (a) enthält, eingespeist wird, und
wobei die Phosphorverbindung (a) eine Oxosäure des Phosphors, eine Oxosäure eines beliebigen alkylierten Phosphors oder ein Salz der Phosphorsäure ist.

2. Verfahren zur Herstellung einer von Keten abgeleiteten acetylierten Verbindung nach Anspruch 1, wobei in Schritt (i) durch Konzentrieren der Phosphorverbindung (a), die in der kondensierten Flüssigkeit enthalten ist, die kondensierte Flüssigkeit in die konzentrierte Flüssigkeit umgewandelt wird, und anschließend die konzentrierte Flüssigkeit in den Reaktor eingespeist wird.

3. Verfahren zur Herstellung einer von Keten abgeleiteten acetylierten Verbindung nach Anspruch 2, wobei die Konzentration der in der konzentrierten Flüssigkeit enthaltenen Phosphorverbindung (a) das 5- bis 20-fache der Konzentration der in der kondensierten Flüssigkeit enthaltenen Phosphorverbindung (a) beträgt.

4. Verfahren zur Herstellung einer von Keten abgeleiteten acetylierten Verbindung nach Anspruch 2 oder 3, wobei ein Verfahren zum Konzentrieren der Phosphorverbindung (a), die in der kondensierten Flüssigkeit enthalten ist, ein Verfahren ausgewählt aus der Gruppe bestehend aus Heizverdampfung, Membrantrennung und elektrochemischer Behandlung ist.

5. Verfahren zur Herstellung einer von Keten abgeleiteten acetylierten Verbindung nach einem der Ansprüche 2 bis 4, wobei in der Stufe (i) eine Flüssigkeit, die durch Zugabe einer Phosphorverbindung (b) zu der konzentrierten Flüssigkeit erhalten wird, in den Reaktor eingespeist wird.

6. Verfahren zur Herstellung einer von Keten abgeleiteten acetylierten Verbindung nach einem der Ansprüche 1 bis 5, wobei das Molverhältnis der Essigsäure zu dem phosphorhaltigen Katalysator 100 bis 2000 beträgt.

## Revendications

1. Procédé de production d'un composé acétylé dérivé de cétène, comprenant :
une étape (i) consistant à réaliser une réaction de décomposition thermique d'acide acétique en présence d'un catalyseur phosphoré dans un réacteur pour produire un gaz de décomposition thermique contenant un cétène,
une étape (ii) consistant à refroidir le gaz de décomposition thermique pour qu'il soit séparé en un composant gazeux contenant du cétène, et un liquide condensé contenant un composé phosphoré (a), et
une étape (iii) consistant à amener le cétène à réagir avec un composé organique différent ayant un groupe carboxyle, un groupe hydroxyle, ou un groupe amino pour produire un composé acétylé dans lequel le groupe carboxyle, le groupe hydroxyle, ou le groupe amino est acétylé,
l'étape (i) incluant : la réalisation de la réaction de décomposition thermique tout en fournissant, dans le réacteur, le liquide condensé contenant le composé phosphoré (a) ou un liquide concentré du liquide condensé contenant le composé phosphoré (a), et
le composé phosphoré (a) étant un oxo-acide du phosphore, un oxo-acide d'un quelconque phosphore alkylé, ou un sel d'acide phosphorique.

2. Procédé de production d'un composé acétylé dérivé de cétène selon la revendication 1, où à l'étape (i), en concentrant le composé phosphoré (a) contenu dans le liquide condensé, le liquide condensé devient le liquide concentré, et le liquide concentré est ensuite fourni dans le réacteur.

3. Procédé de production d'un composé acétylé dérivé de cétène selon la revendication 2, dans lequel une concentration du composé phosphoré (a) contenu dans le liquide condensé est 5 à 20 fois une concentration du composé phosphoré (a) contenu dans le liquide condensé.

4. Procédé de production d'un composé acétylé dérivé de cétène selon la revendication 2 ou 3, dans lequel un procédé de concentration du composé phosphoré (a) contenu dans le liquide condensé est un procédé choisi dans le groupe constitué par de l'évaporation par chauffage, une séparation sur membrane et un traitement électrochimique.

5. Procédé de production d'un composé acétylé dérivé de cétène selon l'une quelconque des revendications 2 à 4, où à l'étape (i), un liquide obtenu par ajout d'un composé phosphoré (b) au liquide concentré est fourni dans le réacteur.

6. Procédé de production d'un composé acétylé dérivé de cétène selon l'une quelconque des revendications 1 à 5, dans lequel un rapport en moles de l'acide acétique au catalyseur phosphoré va de 100 à 2000.
